# EUROPEAN PATENT SPECIFICATION

(11) **EP 1 885 346 B1**
(45) Date of publication and mention of the grant of the patent: **25.11.2009**
(21) Application number: 06763271.1
(22) Date of filing: 24.05.2006
(51) Int. Cl.: A61K 31/047

(54) **USE OF GLYCEROL FOR IMPROVING CARDIAC FUNCTION**
VERWENDUNG VON GLYCERIN ZUR VERBESSERUNG DER HERZFUNKTION
UTILISATION DU GLYCEROL POUR AMELIORER LA FONCTION CARDIAQUE

(30) Priority: 25.05.2005 EP 05291124
(43) Date of publication of application: 13.02.2008
(73) Proprietor: INSTITUT NATIONAL DE LA RECHERCHE AGRONOMIQUE (INRA), 75007 Paris Cédex 07 (FR)
(72) Inventor: GRYNBERG, Alain, F-75015 Paris (FR); GAMBERT, Ségolène, F-94400 Vitry-sur-Seine (FR); HELIES-TOUSSAINT, Cécile, RP 815 F-78008 Versailles Cedex (FR)
(74) Representative: Vialle-Presles, Marie José
(86) International application number: PCT/EP2006/062584
(87) International publication number: WO 2006/125799

(56) References cited:
- US-A- 5 147 650
- US-A1- 2004 192 688

## Description

The present invention relates to the use of glycerol for improving cardiac function.

The capacity of cardiac myocytes to face the ATP production for energy demand is a major determinant of cardiac function. The cardiac myocyte is able to produce energy from a wide range of substrates, including in particular fatty acids (FA) and glucose.

Following their uptake by myocardial cells, FA are imported into the mitochondria by the enzymes carnitine-palmitoyl-transferase (CPT) I and II. Within the mitochondria, they undergo β-oxidation resulting in the formation of acetyl-CoA, which can be used for the formation of ATP through the tricarboxylic acid cycle

In the case of glucose, its entry in the myocardial cells is followed either by its storage as glycogen or by glycolysis which converts it into pyruvate. Pyruvate can enter the mitochondria wherein it is converted into acetyl CoA by pyruvate dehydrogenase.

In the human adult heart, although all substrates compete for energy production, fatty acids represent the main substrate for ATP synthesis and account approximately 70% of the daily oxygen consumption required for cardiac energy production (Opie L., The Heart, Physiology and metabolism, 1991).

Cardiomyocytes are also capable to shift continuously from one source to another, according to the supply availability as controlled by nutritional status, exercise or physiopathological situation.

The production of ATP by the heart is considered to be more than 30 kg per day, whereas ATP store in the cardiac myocyte is very low and allows only 3 to 5 beats. The immediate capacity of the cardiomyocyte to produce energy and to adapt its metabolism to any requirement change is thus one of the most important cardiac functional parameters. Following a carbohydrate-rich meal, insulin favours glucose uptake and the fatty acid oxidation is decreased. In this condition, the contribution of glucose to energy production increases significantly (to 70-80%). Conversely, fasting increases plasma non-esterified fatty acids and cardiac fatty acid uptake and the contribution of fatty acids to energy production increases up to 100%. The same trend occurs after a fatty meal due to plasma triglyceride increase as well as in diabetes or in stress (because of the free fatty acid release from adipocytes after stimulation by catecholamines).

In cardiac myocytes, FAs are required in two main mechanisms: energy production and membrane homeostasis through the phospholipids (PLs) synthesis and remodelling pathways. The synthesis of membrane PLs is a priority that warrants membrane depolarisation, which triggers cardiac contraction. Cardiac myocytes can control FA mitochondrial entry but display a weak ability in controlling FA cellular uptake.

The control of the energy FA/glucose balance appears now clearly as a new strategy in cytoprotection, particularly in diabetes and metabolic diseases and ischemic conditions, all characterised by an excessive consumption of FA and a high FA/glucose balance (up to 100% of FA) (GRYNBERG A., Diabetes Metab., 27, S12-S19, 2001; GRYNBERG, A., Curr. Pharm. Design, 11, 489-509,, 2005).

One of the objectives of cardiac metabolic research is to increase ATP production from glucose and decrease ATP production from fatty acids. One approach consists to lower β-oxidation and control FA management in the myocardium. Several targets have been identified to affect the glucose oxidation to fatty acid β-oxidation balance in the heart (GRYNBERG, 2005, aforementioned).

Etomoxir is an interesting example of these attempts. This selective inhibitor of Carnitine Palmitoyl Transferase I (CPT-I) was developed to decrease glycaemia in the heart by shifting myocardium metabolism to glucose oxidation. Indeed, the drug was shown to improve insulin sensitivity in type 2 diabetic patients (HUBINGER A and al., Horm Metab Res 24, 115-118, 1992) and to increase glucose oxidation in normal and diabetic heart (SCHMITZ and al., Horm Metab Res.,27, 515-522, 1995). The mechanism of action is based on a shift in cardiac fuel utilization that favours the use of glucose as metabolic source in heart, leading to a lowered general glucose availability in the entire organism. Unfortunately, due to the inability of the cardiomyocyte to handle the non-oxidized long chain fatty acids, the chronic treatment with Etomoxir induced cardiac hypertrophy which impaired the development of the drug (RUPP and JACOB, Eur Heart J 13 Suppl D, 56-61, 1992). Decreasing β-oxidation to increase glucose oxidation appears as a positive strategy, but requires a simultaneous handling of the non-oxidized long chain fatty acids.

This is the reason of the success of Trimetazidine (used in more than 90 countries in ischaemia), which reduces β-oxidation and increases the demand of long chain fatty acids for incorporation in membranes by increasing the phospholipid synthesis *in vitro* and *in vivo* (LOPASCHUK and BARR., Mol Cell Biochem, 172, 137-147, 1997, SENTEX and al., Fundam Clin Pharmacol, 15, 255-264, 2001). As a consequence of phospholipid synthesis increase, triacylglycerol synthesis is decreased and the incorporation of long chain fatty acids in the membranes significantly increased, largely enough to decrease the intracellular store of long chain fatty acids available for β-oxidation (see GRYNBERG, 2005, aforementioned for review).

In cardiomyocytes, glycerol is a key metabolite which provides the carbon skeleton for gluconeogenesis, carrying reducing equivalents from the cytosol to mitochondria for oxidative phosphorylation, and acting as the backbone of complex lipids (phospholipids and triacylglycerols) (LIN, Annu Rev Biochem 46: 765-795, 1977). Endogenous glycerol from an extracellular source is phosphorylated to glycerol-3-phosphate and then acylated to enter in the phospholipids synthesis pathways. Although the metabolic pathways for ATP production from glycerol as well as the pathways leading to glycogen do theoretically exist in the heart, their functionality has not been demonstrated until now.

The inventors have reported earlier that the increased phospholipid synthesis due to Trimetazidine is associated with a significant increase in cellular glycerol uptake (SENTEX and al, 2001, aforementioned).

The inventors have further studied in isolated neonatal rat cardiomyocytes the influence of glycerol concentration on glycerol uptake and on the balance between phospholipids and triacylglycerol synthesis (GAMBERT and al., Archives des maladies du coeur et des vaisseaux . Abstract 20-4, 2004). They have observed that glycerol uptake increases with extracellular glycerol concentration from 80 µM to 660 µM.

The synthesis of complex lipids was increased in parallel to glycerol uptake, although in a different way for phospholipids and triacylglycerols. While the PL concentration increased progressively as a function of the extracellular glycerol concentration, the triacylglycerol concentration only increased at higher glycerol concentrations. When Trimetazidine was added, glycerol uptake was increased. Trimetazidine showed no significant effect on triacylglycerol synthesis. As for phospholipids, Trimetazidine increased their synthesis only at high glycerol concentrations.

These results show that in cell cultures, under conditions of constant energy demand, cardiac glycerol uptake increase the fatty acid demand for complex lipids synthesis. At low glycerol concentrations, the requirements of PL *de novo* synthesis are not fully satisfied whereas at higher glycerol concentrations, the available intracellular glycerol overflows these requirements. They suggest that there is a difference in cardiac membrane homeostasis according to the glycerol supply and that cardiac membrane turnover is a priority for the cell as compared to TAG synthesis.

However the results reported above were obtained on a cell culture model, that does not reflect accurately the complex physiology of heart, and in particular does not allow to predict the myocardium metabolic responses to the variations in energy demand occurring continuously in the heart *in vivo* as a consequence of the variations in cardiac rate.

The inventors have now tested the effects of glycerol on myocardium energy metabolism in an *in vitro*/*ex vivo* model of isolated working rat heart.

They found that in these conditions, glycerol is not only involved in the synthesis of glycerolipids, but is also a substrate involved in energy production, through the mitochondrial production of CO₂.

Further, this production is significantly increased with the energy demand independently of the circulating glycerol concentration.

They also found that at high circulating glycerol concentration, glycerol cardiac uptake is controlled by cardiac energy demand, i.e. the increase in cardiac rate results in a significant increase in glycerol uptake, while at low glycerol concentration, glycerol uptake is not significantly modified by the cardiac rate.

At high circulating glycerol concentration, the intracellular available glycerol is oriented towards the synthesis of glycerolipids, with a preferential incorporation of saturated fatty acids in the complex lipid pools (membrane and storage). This increase in the synthesis of glycerolipids is not correlated with an increase in the fatty acids uptake by the heart, showing that it occurs at the expense of β-oxydation of fatty acids. The inventors have confirmed this hypothesis by demonstrating that when the β-oxidation of palmitate is high, the increase of glycerol induce a significant decrease in β-oxidation.

These results clearly demonstrate the importance of glycerol in regulating the cardiac metabolic pathways and energy balance, according to energy demand. This allows to propose its use for the regulation of cardiac metabolism, and for cardiac cytoprotection.

Accordingly, an object of the present invention is the use of glycerol as an active principle for preparing a composition reducing myocardial β-oxydation of fatty acids, useful for treating or preventing cardiovascular diseases. Said cardiovascular diseases include in particular myocardial ischemic conditions resulting from an excessive β-oxydation of fatty acids. By way of non-limitative examples, one can mention: ischemic cardiomyopathies (angina pectoris, myocardial infarct), cardiac consequences of diabetes, cardiac hypertrophy and heart failure.

According to a preferred embodiment of the invention, said composition is formulated for oral administration of glycerol, advantageously in the form of a food, beverage, or food complement.

However, the parenteral route can also be used, including for instance, intravenous injection.

By way of illustration:
- in the case of oral administration, glycerol is administered preferably at a dose of from about 1 to 2 g per kg body weight per day for a human adult.
- in the case of parenteral administration, glycerol can be administered as a 10% solution in saline buffer (NaC1) at a dose of 1g/kg body weight/day for a human adult.

It is to be understood that the dosage indicated above are given by way of example only, and that the specific dosage of glycerol to be administered, as well as the interval between administrations and the duration of treatment depend on factors such as the nature and the severity of the disease to be treated and the age and the condition of the patient. They can easily be determined by the skilled practitioner on the basis of the disclosure provided herein.

According to another preferred embodiment of the invention, said composition may include one or several additional active principles. For example, glycerol may be used in conjunction with any diet or any parenteral clinical nutrition or treatment, such as glucose-insuline-potassium perfusion used in the acute phase of ischaemia.

The present invention will be understood more clearly from the further description which follows, which refers to non-limiting examples illustrating the effects of glycerol on myocardium energy metabolism in an isolated working heart.

### EXAMPLE 1: INVOLVEMENT OF GLYCEROL IN THE ENERGY PRODUCTION IN THE HEART

### Isolated heart experiments

The investigations were carried out on a perfused rat heart model (LOPASCHUK and BARR., 1997, aforementioned, SENTEX et al, 2001, aforementioned, FOLCH et al., J Biol Chem, 226, 497-509, 1957). Male Wistar rats (280-330 g) were anaesthetised with sodium pentobarbital (60 mg per kg, i.p.) and heparin was intravenously injected (500 IU/kg). After 1 min, the hearts were excised and placed in a cold (4°C) perfusion buffer awaiting contractions ceased. The heart was then immediately cannulated through the aorta and perfused according to the Langendorff method at a constant perfusion pressure of 80 cm of water (8 kPa). The perfusion fluid consisted of a modified Krebs-Henseleit bicarbonate buffer (mM concentrations: NaCl 118, NaHCO₃ 25, MgSO₄ 1.2, KH₂PO₄ 1.2, KCl 4.7, glucose 5.5 and CaCl₂ 2.0).

Two additional solutions, supplemented with 2 glycerol concentrations : 0.33 mM representative of fasting state(<0.5 mM), or 3.33 mM representative of fed state (>3 mM), were prepared from this basal solution.

Before use, all solutions were filtered through a 0.8 µm filter (Millipore, Saint Quentin en Yvelines, France) to remove any particulate contaminants. The perfusion fluid was gassed with 95% oxygen-5% carbon dioxide (pH 7.3-7.5 at 37°C). The intraventricular pressure and the heart rate were recorded together with ECG. Coronary flow was measured by sequentially collecting the effluent. After stabilization, the perfusion solution was turned to the same solution containing [¹⁴C]-glycerol (1µCi/mL).

The effect of energy demand on glycerol metabolism was investigated by pacing the heart at either 350 beats/min (normal rat rate) or 450 beats/min.

The cardiac capacity to use glycerol as a metabolic substrate in energy production, according to glycerol availability was evaluated using ¹⁴C-radiolabelled glycerol.

The direct measurement of mitochondrial energy production was made by quantitatively collecting ¹⁴CO₂ produced from the perfused hearts. After stabilization of cardiac function, the gas mixture (95% O₂-5% CO₂) outflow containing the ¹⁴CO₂ is allowed to bubble through a hyamine peroxide solution. The hyamine peroxide was collected every 10 minutes and the radioactivity evaluated.

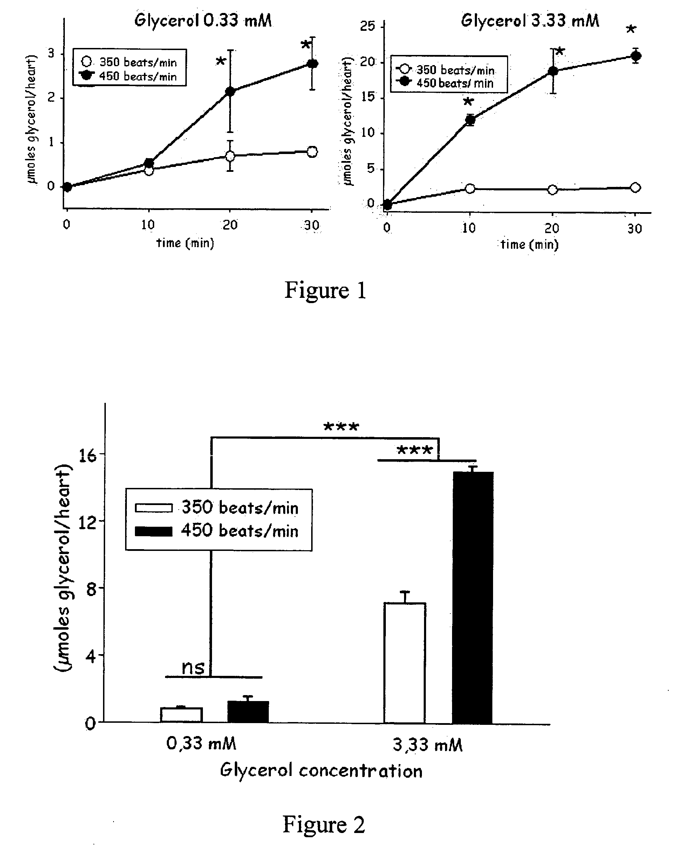

The results are illustrated by Figure 1.

These results show the metabolic implication of glycerol in cardiac energy production through the mitochondrial production of ¹⁴CO₂. This production is significantly increased with the energy demand (from 350 to 450 beats/min) whatever the circulating glycerol concentration.

### EXAMPLE 2- EFFECT OF CARDIAC ENERGY DEMAND AND CIRCULATING GLYCEROL CONCENTRATION ON GLYCEROL UPTAKE AND ITS INCORPORATION IN GLYCEROLIPIDS.

The experiments were performed using the perfusion conditions described in Example 1.

At the end of the perfusion, the heart was washed out from the remaining radiolabelled medium by 10 mL of Krebs-Henseleit solution injected through the canula. The hearts were homogenized in 30 mL chloroform/methanol (2/1, v/v) according to FOLCH (1957, aforementioned). The radioactivity of the homogenate was evaluated.

A 0.73% NaCl solution was added to the homogenate to cause phase separation. The radioactivity was determined in samples of the aqueous and organic fractions, and the phospholipids (PL) were separated from non-phosphorous lipids (NPL) on silica cartridges (Supelco, Sigma) and the radioactivity of each class was evaluated.

### Glycerol uptake:

The glycerol uptake was evaluated from the total radioactivity of the homogenate.

The results are illustrated by Figure 2.

These results show that at low glycerol concentration (0,33 mM) the glycerol uptake is not significantly modified by the increase in rate. However at high glycerol concentration (3.33 mM) the increased energetic demand results in a significant increase in glycerol uptake.

### Incorporation of glycerol in glycerolipids:

The fate of intracellular glycerol was evaluated in these rat hearts by analysing the radioactivity incorporated into phospholipids (PL) and non-phosphorous lipids (NPL), which in the heart represent mainly the triacylglycerol pool.

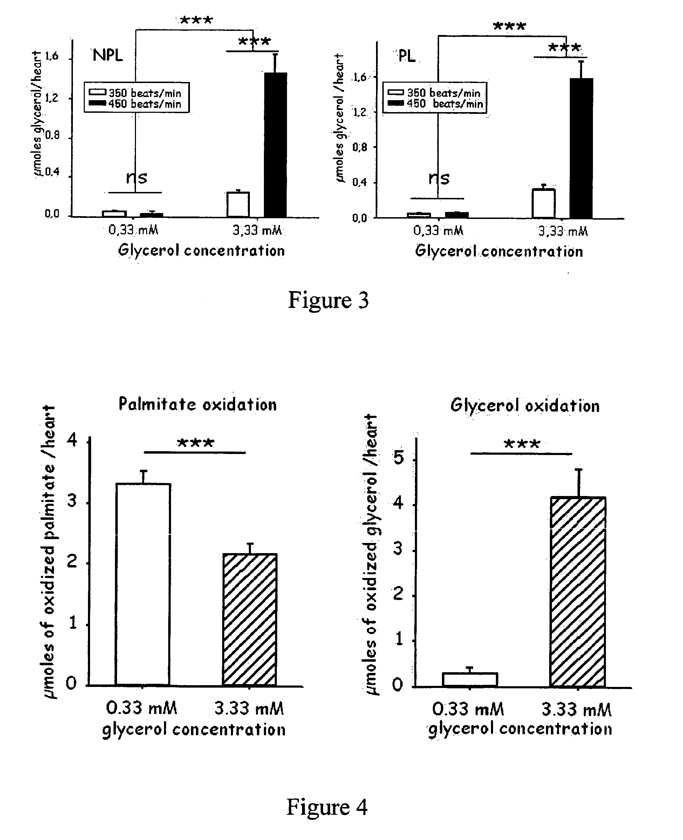

The results are illustrated by Figure 3.

These results show that the increase in rate does not influence the synthesis of glycerolipids at low glycerol concentration (0.33 mM) whereas at high glycerol concentration (3,33 mM), the glycerol is involved in the synthesis of both membrane phospholipids and storage triacylglycerols.

### EXEMPLE 3: EFFECT OF CARDIAC ENERGY DEMAND AND CIRCULATING GLYCEROL CONCENTRATION ON PALMITATE INCORPORATION.

The influence of cardiac energy demand and circulating glycerol concentration on fatty acid metabolism was evaluated in the heart by following the intracellular palmitate fate with [³H]-palmitate.

The experiments were performed using the perfusion conditions described in Example 1, except that a perfusion solution supplemented with palmitate (0.4 mM) was used.

To prepare palmitate-enriched solution, 43.5g of albumin (fraction bovine, Sigma) was dissolved in 350 mL Krebs-Henselheit buffer. Palmitate was then slowly added to this albumin solution and mixed. The albumin-palmitate mixture was dialysed against Krebs-Henselheit solution at + 4°C (three time 12 h dialysis in 10 basal solution volume). For the final preparation of the palmitate-enriched fluid, 93.5 mL of concentrated solution were incorporated in 2 L of Krebs-Henselheit Radioactive solution was prepared in the same conditions with addition of [³H]-palmitate (1µCi/mL). The perfusion solution also contained [¹⁴C]-glycerol to follow glycerol oxidation in the same heart.

The oxidations of palmitate and glycerol were evaluated simultaneously using as tracers [³H]-palmitate and [¹⁴C]-GLYCEROL, respectively. At the end of the perfusion, the oxidized substrates were determined by measuring the radioactivity rejected by the heart in the perfusion medium as [³H]₂O and [¹⁴C]O₂, respectively. The results are shown in figure 4.

These results show that when the extracellular glycerol concentration increased, the glycerol oxidation increased, whereas the FA β-oxidation was reduced. These result are consistent with the result showed in example 2, showing that increased glycerol supply results in increased synthesis of complex lipids, which requires the redirection of FAs to membrane synthesis and thus decreases the FA pool available for β-oxidation.

## Claims

1. Use of glycerol as an active principle for preparing a composition reducing myocardial β-oxydation of fatty acids, useful for treating or preventing a cardiovascular disease.

2. The use of claim 1, wherein said disease is selected among ischemic cardiomyopathies (angina pectoris, myocardial infarct), cardiac consequences of diabetes, cardiac hypertrophy and heart failure.

3. The use of any of claims 1 or 2 wherein said composition is formulated for oral administration.

4. The use of claim 3, wherein said composition is in the form of a food, beverage, or food complement.

5. The use of claim 4, wherein said composition is formulated for parenteral administration.

## Patentansprüche

1. Verwendung von Glycerin als Wirkstoff zur Herstellung einer Zusammensetzung, die eine myokardiale β-Oxidation von Fettsäuren verringert, die zur Behandlung oder Prävention einer kardiovaskulären Erkrankung nützlich ist.

2. Verwendung nach Anspruch 1, wobei die Erkrankung aus ischämischen Kardiomyopathien (Angina pectoris, Myokardinfarkt), Folgen von Diabetes für das Herz, kardialer Hypertrophie und Herz(kraft)versagen ausgewählt ist.

3. Verwendung nach einem der Ansprüche 1 oder 2, wobei die Zusammensetzung zur oralen Verabreichung formuliert ist.

4. Verwendung nach Anspruch 3, wobei die Zusammensetzung in der Form eines Nahrungsmittels, eines Getränks oder einer Nahrungsergänzung ist.

5. Verwendung nach Anspruch 4, wobei die Zusammensetzung zur parenteralen Verabreichung formuliert ist.

## Revendications

1. Utilisation de glycérol comme principe actif pour la préparation d'une composition réduisant la β-oxydation myocardique des acides gras, utile pour traiter ou prévenir une maladie cardiovasculaire.

2. Utilisation selon la revendication 1, dans laquelle ladite maladie est choisie parmi les cardiomyopathies ischémiques (angine de poitrine, infarctus du myocarde), les conséquences cardiaques du diabète, l'hypertrophie cardiaque et l'insuffisance cardiaque.

3. Utilisation selon l'une quelconque des revendications 1 ou 2, dans laquelle ladite composition est formulée pour une administration orale.

4. Utilisation selon la revendication 3, dans laquelle ladite composition est sous la forme d'un aliment, d'une boisson ou d'un complément alimentaire.

5. Utilisation selon la revendication 4, dans laquelle ladite composition est formulée pour une administration parentérale.
